# FASCICULE DE BREVET EUROPEEN

(11) **EP 0 528 968 B1**
(45) Date de publication et mention de la délivrance du brevet: **02.02.1994**
(21) Numéro de dépôt: 91910359.8
(22) Date de dépôt: 15.05.1991
(51) Int. Cl.: C07D 277/82, A61K 31/425

(54) **DERIVES D'(ALKYLTHIO-3 PROPYL)-3 BENZOTHIAZOLINE, LEUR PREPARATION ET LES MEDICAMENTS LES CONTENANT**
(ALKYLTHIO-3-PROPYL)-3-BENZOTHIAZOLINDERIVATE, IHRE HERSTELLUNG UND DIESE ENTHALTENDE ARZNEIMITTEL
(3-ALKYLTHIO PROPYL)-3 BENZOTHIAZOLINE DERIVATIVES, PREPARATION THEREOF AND DRUGS CONTAINING SAID DERIVATIVES

(30) Priorité: 18.05.1990 FR 9006277
(43) Date de publication de la demande: 03.03.1993
(73) Titulaire: RHONE-POULENC RORER S.A., 92165 Antony Cédex (FR)
(72) Inventeur: GUEREMY, Claude, F-78800 Houilles (FR); JIMONET, Patrick, F-78450 Villepreux (FR)
(74) Mandataire: Savina, Jacques (FR)
(86) Numéro de dépôt international: FR9100390
(87) Numéro de publication internationale: WO9117984

(56) Documents cités:
- EP-A- 0 050 551
- EP-A- 0 282 971
- EP-A- 0 305 276
- EP-A- 0 305 277
- DE-A- 2 834 852

## Description

La présente invention concerne des composés de formule : 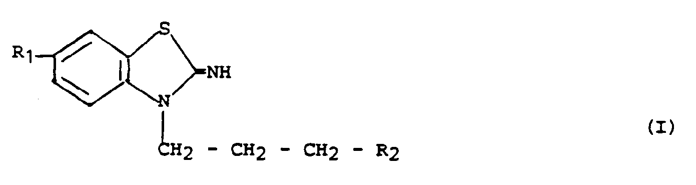
leurs sels, leurs procédés de préparation et les médicaments les contenant.

Dans la formule (I) :
- R₁ représente un radical polyfluoroalcoxy,
- R₂ représente un radical alkylthio, alkylsulfinyle ou alkylsulfonyle.

Dans les définitions qui précèdent et celles qui seront citées ci-après, les radicaux alkyle et alcoxy et les portions alkyle et alcoxy contiennent 1 à 4 atomes de carbone en chaîne droite ou ramifiée.

Les radicaux polyfluoroalcoxy sont de préférence les radicaux trifluorométhoxy, pentafluoroéthoxy, trifluoro-2,2,2 éthoxy, ou tétrafluoro-1,1,2,2 éthoxy.

L'invention concerne également les sels d'addition des composés de formule (I) avec les acides minéraux ou organiques.

Selon l'invention, les composés de formule (I) pour lesquels R₂ représente un radical alkylthio, peuvent être préparés par action d'un dérivé de formule :

R₃ - S - Na (II)

dans laquelle R₃ représente un radical alkyle sur un dérivé de formule : 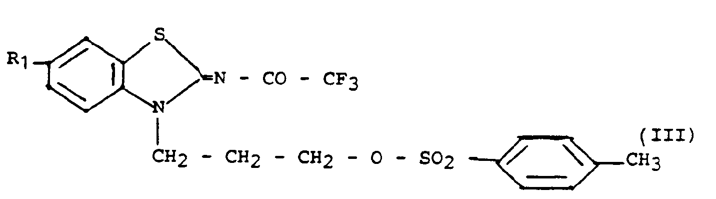
dans laquelle R₁ a les mêmes significations que dans la formule (I).

Cette réaction s'effectue, généralement, au sein d'un solvant organique inerte tel qu'un alcool (méthanol, éthanol par exemple), à une température comprise entre 20°C et la température d'ébullition du milieu réactionnel.

Les dérivés de formule (III) peuvent être obtenus par action de chlorure de p-toluène sulfonyle sur un dérivé de formule : 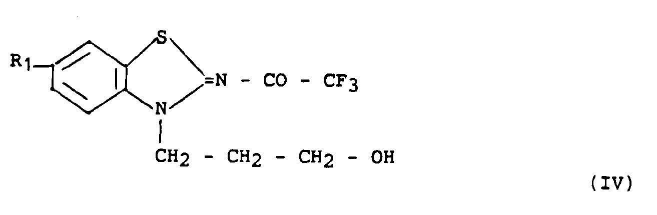
dans laquelle R₁ a les mêmes significations que dans la formule (I).

Cette réaction s'effectue, de préférence, au sein de la pyridine, à une température comprise entre 0°C et 10°C.

Les dérivés de formule (IV) peuvent être obtenus par action de trifluoroacétate d'éthyle sur un dérivé de formule : 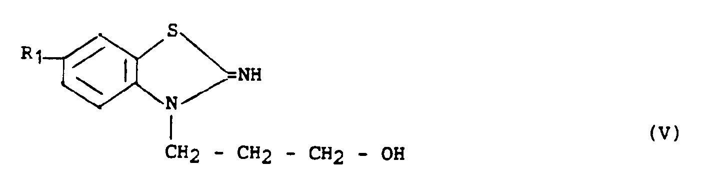
dans laquelle R₁ a les mêmes significations que dans la formule (I).

Cette réaction s'effectue, généralement, au sein d'un solvant inerte tel qu'un alcool (méthanol, éthanol par exemple), en présence d'une trialkylamine, à une température voisine de 20°C.

Les dérivés de formule (V) peuvent être obtenus par action de bromo-3 propanol sur un dérivé de formule : 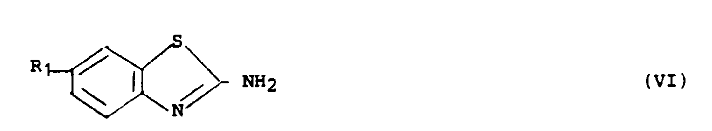
dans laquelle R₁ a les mêmes significations que dans la formule (I).

Cette réaction s'effectue, généralement, au sein d'un solvant inerte tel qu'un alcool (éthanol, propanol-2 par exemple), à la température d'ébullition du solvant.

Les dérivés de formule (II) peuvent être obtenus par application ou adaptation de la méthode décrite par L.M. YAGUPOL'SKII et coll., Zh, Obshch. Khim., 33(7), 2301-7 (1963) (Chem. Abst., vol 60, 692a-f (1964)).

Les composés de formule (I) pour lesquels R₂ représente un radical alkylsulfinyle ou alkylsulfonyle peuvent être préparés par oxydation d'un dérivé de formule (I) correspondant pour lequel R₂ représente un radical alkylthio éventuellement suivie d'une hydrolyse pour libérer la fonction imino.

L'oxydation en alkylsulfinyle est effectuée généralement au moyen d'acide m-chloroperbenzoïque, au sein d'un solvant inerte tel que l'eau, le dioxane ou un mélange de ces solvants, à une température voisine de 20°C.

L'oxydation en alkylsulfonyle est généralement effectuée au moyen d'acide m-chloroperbenzoïque en excès, au sein d'un solvant chloré (chloroforme, dichlorométhane par exemple), à la température d'ébullition du solvant.

L'hydrolyse s'effectue généralement au moyen d'un acide tel que l'acide chlorhydrique, au sein d'un solvant inerte tel que le dioxane, à la température d'ébullition du milieu réactionnel.

Les mélanges réactionnels obtenus par les divers procédés décrits précédemment sont traités suivant des méthodes classiques physiques (évaporation, extraction, distillation, cristallisation, chromatographie...) ou chimiques (formation de sels...).

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés sont actifs vis-à-vis des convulsions induites par le glutamate et sont donc utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyotrophique latérale et l'atrophie olivopontocérébelleuse.

L'activité des composés de formule (I) vis-à-vis des convulsions induites par le glutamate a été déterminée selon une technique inspirée de celle de I.P. LAPIN, J. Neural. Transmission, vol. 54, 229-238 (1982) ; l'injection du glutamate par voie intracérébroventriculaire étant effectuée selon une technique inspirée de celle de R. CHERMAT et P. SIMON, J. Pharmacol. (Paris), vol. 6, 489-492 (1975). Leur DE₅₀ est généralement égale ou inférieure à 10 mg/kg.

Les composés de formule (I) présentent une toxicité faible. Leur DL₅₀ est supérieure à 15 mg/kg par voie IP chez la souris.

D'un intérêt particulier sont les composés suivants :
- imino-2 (méthylthio-3 propyl)-3 trifluorométhoxy-6 benzothiazoline
- imino-2 (méthylsulfinyl-3 propyl)-3 trifluorométhoxy-6 benzothiazoline-(RS)
- imino-2 (méthylsulfonyl-3 propyl)-3 trifluorométhoxy-6 benzothiazoline.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, isothionate, théophillineacétate, salicylate, phénolphtalinate, méthylène-bis-β-oxynaphtoate, chlorhydrate, sulfate, nitrate et phosphate.

Les exemples suivants, donnés à titre non limitatif, montrent comment l'invention peut être mise en pratique.

### EXEMPLE 1

3,88 g de p-toluènesulfonate de (trifluoroacétylimino-2 trifluorométhoxy-6 benzothiazolinyl-3)-3 propyle et 1,03 g de méthanethiolate de sodium dans 20 cm³ d'éthanol absolu sont chauffés à 60°C pendant 6 heures. Après refroidissement à une température voisine de 20°C, le milieu réactionnel est concentré à sec sous pression réduite (20 mm de mercure ; 2,7 kPa). Le résidu huileux est repris dans 50 cm³ d'eau distillée et la phase organique extraite par 50 cm³ d'éther éthylique. Après séchage sur sulfate de magnésium et concentration à sec sous pression réduite, on obtient 1,88 g d'imino-2 (méthylthio-3 propyl)-3 trifluorométhoxy-6 benzothiazoline que l'on transforme en chlorhydrate fondant à 181°C.

Le p-toluènesulfonate de (trifluoroacétylimino-2 trifluorométhoxy-6 benzothiazolinyl-3)-3 propyle peut être préparé selon le procédé suivant : 11 g de (trifluoroacétylimino-2 trifluorométhoxy-6 benzothiazolinyl-3)-3 propanol sont ajoutés progressivement à 10,8 g de chlorure de p-toluènesulfonyle en solution dans 200 cm³ de pyridine refroidie à 0°C. La réaction est poursuivie 1 heure à 5°C, puis le milieu réactionnel est gardé au froid (6-7°C) pendant 15 heures. Après addition à 2 litres d'eau distillée, extraction par 200 cm³ d'acétate d'éthyle, lavage par 2 fois 50 cm³ d'acide chlorhydrique 1N, séchage sur sulfate de magnésium et concentration à sec sous pression réduite, on obtient 6,7 g de p-toluènesulfonate de (trifluoroacétylimino-2 trifluorométhoxy-6 benzothiazolinyl-3)-3 propyle fondant à 139°C.

Le (trifluoroacétylimino-2 trifluorométhoxy-6 benzothiazolinyl-3)-3 propanol peut être préparé de la façon suivante : 24,8 g d'(imino-2 trifluorométhoxy-6 benzothiazolinyl-3)-3 propanol, 14,2 g de trifluoroacétate d'éthyle et 8,6 g de triéthylamine sont agités dans 250 cm³ d'éthanol absolu pendant 24 heures à une température voisine de 20°C. Le milieu réactionnel est alors refroidi à 0-5°C et le précipité formé filtré et séché. On obtient 28,2g de (trifluoroacétylimino-2 trifluorométhoxy-6 benzothiazolinyl-3)-3 propanol fondant à 144°C.

L'(imino-2 trifluorométhoxy-6 benzothiazolinyl-3)-3 propanol peut être préparé selon le procédé suivant : 82 g d'amino-2 trifluorométhoxy-6 benzothiazole et 65 cm³ de bromo-3 propanol dans 25 cm³ d'éthanol absolu sont chauffés pendant 72 heures à ébullition. Le mélange est ensuite refroidi à une température voisine de 20°C. L'huile obtenue est reprise dans 1 litre d'eau distillée et la phase organique extraite par 3 fois 200 cm³ de dichlorométhane. Après séchage sur sulfate de magnésium et concentration à sec sous pression réduite, le produit brut est purifié par chromatographie sur colonne de silice avec de l'acétate d'éthyle comme éluant. On obtient 25 g d'(imino-2 trifluorométhoxy-6 benzothiazolinyl-3)-3 propanol fondant à 106°C.

### EXEMPLE 2

A 4,2 g d'imino-2 (méthylthio-3 propyl)-3 trifluorométhoxy-6 benzothiazoline en solution dans 100 cm³ d'un mélange d'eau et de dioxane (40-60 en volumes), on ajoute à une température voisine de 20°C, 2,5 g d'acide m-chloroperbenzoïque à 90 % en environ 15 minutes. Le milieu réactionnel est agité à la même température pendant 72 heures. Après addition à 500 cm³ d'eau distillée et neutralisation par de la soude 1N, le milieu réactionnel est extrait par du dichlorométhane. Après séchage sur sulfate de magnésium et concentration à sec sous pression réduite puis purification par chromatographie sur colonne de silice avec un mélange d'acétate d'éthyle et de méthanol (80-20 en volumes) comme éluant, on obtient 1,2 g d'imino-2 (méthylsulfinyl-3 propyl)-3 trifluorométhoxy-6 benzothiazoline-(RS) fondant à 114°C.

### EXEMPLE 3

0,7 g de (chloro-3 benzoylimino)-2 (méthylsulfonyl-3 propyl)-3 trifluorométhoxy-6 benzothiazoline dans 100 cm³ de dioxane est traité par 5 cm³ d'acide chlorhydrique à 37 % pendant 48 heures à ébullition. Le milieu réactionnel est refroidi à une température voisine de 20°C et concentré à sec sous pression réduite puis le résidu est repris dans 150 cm³ d'eau distillée. Après neutralisation par de la soude 1N, extraction par de l'acétate d'éthyle et formation de l'oxalate dans l'acétone, on obtient 0,2 g d'oxalate d'imino-2 (méthylsulfonyl-3 propyl)-3 trifluorométhoxy-6 benzothiazoline fondant à 180°C.

La (chloro-3 benzoylimino)-2 (méthylsulfonyl-3 propyl)-3 trifluorométhoxy-6 benzothiazoline peut être préparée de la façon suivante : à 3,9 g d'imino-2 (méthylthio-3 propyl)-3 trifluorométhoxy-6 benzothiazoline en solution dans 100 cm³ de dichlorométhane agités à une température voisine de 20°C, on ajoute 5,2 g d'acide m-chloroperbenzoïque à 75 % en environ 10 minutes. Le milieu réactionnel est porté à ébullition pendant 18 heures. Après refroidissement à une température voisine de 20°C, le précipité est filtré, lavé par 50 cm³ de dichlorométhane et le filtrat concentré à sec sous pression réduite après lavage avec de la soude 1N. L'huile obtenue est cristallisée dans l'acétate d'éthyle. On obtient ainsi 0,6 g de (chloro-3 benzoylimino)-2 (méthylsulfonyl-3 propyl)-3 trifluorométhoxy-6 benzothiazoline fondant à 188°C.

La présente invention concerne également les médicaments constitués par au moins un composé de formule (I) ou un sel d'un tel composé à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice.

Ces compositions peuvent également comprendre des substances autres que les diluants, par exemple un ou plusieurs lubrifiants tels que le stéarate de magnésium ou le talc, un colorant, en enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles dans le traitement et la prévention des phénomènes convulsifs, des troubles schizophréniques et notamment des formes déficitaires de la schizophrénie, des troubles du sommeil, des phénomènes liés à l'ischémie cérébrale et des affections neurologiques où le glutamate peut être impliqué telles que la maladie d'Alzheimer, la maladie d'Huntington, la sclérose amyotrophique latérale et l'atrophie olivopontocérébelleuse.

Les doses dépendent de l'effet recherché, de la durée du traitement et de la voie d'administration utilisée ; elles sont généralement comprises entre 30 et 300 mg par jour par voie orale pour un adulte avec des doses unitaires allant de 10 à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- imino-2 (méthylthio-3 propyl)-3 trifluorométhoxy-6 benzothiazoline 50 mg
- cellulose 18 mg
- lactose 55 mg
- silice colloïdale 1 mg
- carboxyméthylamidon sodique 10 mg
- talc 10 mg
- stéarate de magnésium 1 mg

### EXEMPLE B

On prépare, selon la technique habituelle, des comprimés dosés à 50 mg de produit actif ayant la composition habituelle :
- imino-2 (méthylsulfinyl-3 propyl)-3 trifluorométhoxy-6 benzothiazoline 50 mg
- lactose 104 mg
- cellulose 40 mg
- polyvidone 10 mg
- carboxyméthylamidon sodique 22 mg
- talc 10 mg
- stéarate de magnésium 2 mg
- silice colloïdale 2 mg
- mélange d'hydroxyméthylcellulose, glycérine, oxyde de titane (71-3, 5-24, 5) q.s.p. 1 comprimé pelliculé terminé à 245 mg

### EXEMPLE C

On prépare une solution injectable contenant 10 mg de produit actif ayant la composition suivante :
- imino-2 (méthylsulfonyl-3 propyl)-3 trifluorométhoxy-6 benzothiazoline 10 mg
- acide benzoïque 80 mg
- alcool benzylique 0,06 cm³
- benzoate de sodium 80 mg
- éthanol à 95 % 0,4 cm³
- hydroxyde de sodium 24 mg
- propylène glycol 1,6 cm³
- eau q.s.p 4 cm³

## Revendications (Revendications pour l'(les) Etat(s) contractant(s) suivant(s): AT, BE, CH, DE, DK, FR, GB, IT, LI, LU, NL, SE)

1. Composés de formule : 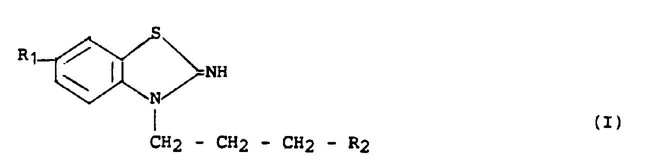 dans laquelle R₁ représente un radical polyfluoroalcoxy, et R₂ représente un radical alkylthio, alkylsulfinyle ou alkylsulfonyle, étant entendu que les portions alkyle et alcoxy contiennent 1 à 4 atomes de carbone en chaîne droite ou ramifiée, ainsi que leurs sels avec des acides minéraux ou organiques.

2. Composés de formule (I) selon la revendication 1 pour lesquels R₁ représente un radical trifluorométhoxy, pentafluoroéthoxy, trifluoro-2,2,2 éthoxy ou tétrafluoro-1,1,2,2 éthoxy.

3. Procédé de préparation des composés de formule (I) selon la revendication 1 pour lesquels R₂ représente un radical alkylthio caractérisé en ce que l'on fait réagir un dérivé de formule :
R₃ - S - Na (II)
dans laquelle R₃ représente un radical alkyle sur un dérivé de formule : 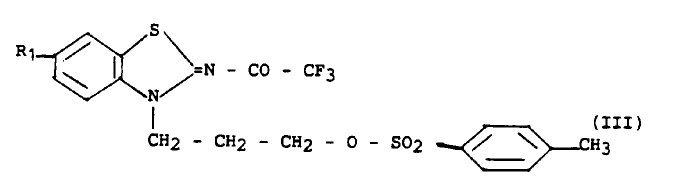 dans laquelle R₁ a les mêmes significations que dans la revendication 1 puis isole le produit et le transforme éventuellement en sel d'addition avec un acide organique ou minéral.

4. Procédé de préparation des composés de formule (I) selon la revendication 1 pour lesquels R₂ représente un radical alkylsulfinyle ou alkylsulfonyle caractérisé en ce que l'on oxyde un dérivé de formule (I) correspondant pour lequel R₂ représente un radical alkylthio et éventuellement hydrolyse puis isole le produit obtenu et le transforme éventuellement en sel d'addition avec un acide organique ou minéral.

5. Médicaments caractérisés en ce qu'ils contiennent comme principe actif au moins un composé selon la revendication 1 ou un sel d'un tel composé.

6. Médicaments selon la revendication 5 pour le traitement des affections où le glutamate est impliqué.

## Revendications (Revendications pour l'(les) Etat(s) contractant(s) suivant(s): ES, GR)

1. Procédé de préparation des composés de formule : 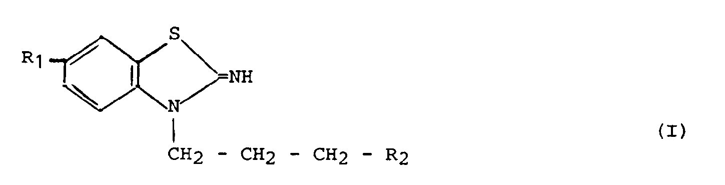 dans laquelle R₁ représente un radical polyfluoroalcoxy, et R₂ représente un radical alkylthio, alkylsulfinyle ou alkylsulfonyle, étant entendu que les portions alkyle et alcoxy contiennent 1 à 4 atomes de carbone en chaîne droite ou ramifiée, ainsi que leurs sels avec des acides minéraux ou organiques, caractérisé en ce que
A. pour la préparation des composés de formule (I) pour lesquels R₂ représente un radical alkylthio on fait réagir un dérivé de formule :
R₃ - S - Na (II)
dans laquelle R₃ représente un radical alkyle sur un dérivé de formule : 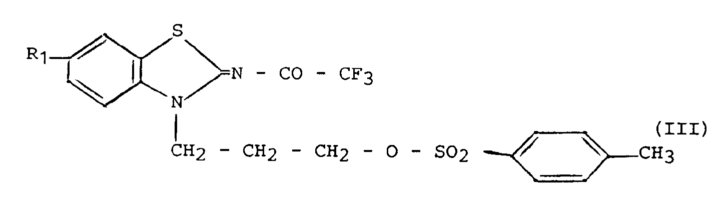 dans laquelle R₁ a les mêmes significations que dans la formule (I) puis isole le produit et le transforme éventuellement en sel d'addition avec un acide organique ou minéral,
B. pour la préparation des composés de formule (I) pour lesquels R₂ représente un radical alkylsulfinyle ou alkylsulfonyle on oxyde un dérivé de formule (I) correspondant pour lequel R₂ représente un radical alkylthio et éventuellement hydrolyse puis isole le produit obtenu et le transforme éventuellement en sel d'addition avec un acide organique ou minéral.

2. Procédé selon la revendication 1 pour la préparation des composés de formule (I) pour lesquels R₁ représente un radical trifluorométhoxy, pentafluoroéthoxy, trifluoro-2,2,2 éthoxy ou tétrafluoro-1,1,2,2 éthoxy.

## Patentansprüche (Patentansprüche für folgende(n) Vertragsstaat(en): AT, BE, CH, DE, DK, FR, GB, IT, LI, LU, NL, SE)

1. Verbindungen der Formel 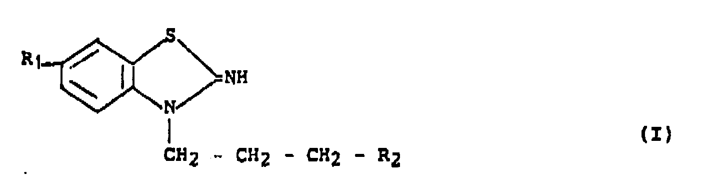 worin R₁ einen Polyfluoralkoxyrest bedeutet und R₂ einen Alkylthio-, Alkylsulfinyl- oder Alkylsulfonylrest darstellt, wobei die Alkyl- und Alkoxyteile 1 bis 4 Kohlenstoffatome in gerader oder verzweigter Kette enthalten, sowie ihre Salze mit organischen oder Mineralsäuren.

2. Verbindungen der Formel (I) gemäß Anspruch 1, worin R₁ einen Trifluormethoxy-, Pentafluorethoxy-, 2,2,2-Trifluorethoxy- oder 1,1,2,2-Tetrafluorethoxyrest bedeutet.

3. Verfahren zur Herstellung der Verbindungen der Formel (I) gemäß Anspruch 1, worin R₂ einen Alkylthiorest darstellt, dadurch gekennzeichnet, daß man ein Derivat der Formel
R₃ - S - Na (II)
worin R₃ einen Alkylrest bedeutet, auf ein Derivat der Formel 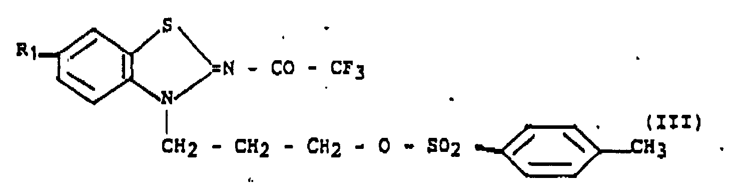 worin R₁ die gleichen Bedeutungen wie in Anspruch 1 hat, einwirken läßt, das Produkt dann isoliert und es gegebenenfalls in ein Additionssalz mit einer organischen oder Mineralsäure überführt.

4. Verfahren zur Herstellung der Verbindungen der Formel (I) gemäß Anspruch 1, worin R₂ einen Alkylsulfinyl- oder Alkylsulfonylrest bedeutet, dadurch gekennzeichnet, daß man ein entsprechendes Derivat der Formel (I), worin R₂ einen Alkylthiorest bedeutet, oxidiert und gegebenenfalls hydrolysiert, dann das erhaltene Produkt isoliert und es gegebenenfalls in ein Additionssalz mit einer organischen oder Mineralsäure überführt.

5. Arzneimittel, dadurch gekennzeichnet, daß sie als Wirkstoff mindestens eine Verbindung gemäß Anspruch 1 oder ein Salz einer solchen Verbindung enthalten.

6. Arzneimittel gemäß Anspruch 5 zur Behandlung von Erkrankungen, worin das Glutamat einbezogen ist.

## Patentansprüche (Patentansprüche für folgende(n) Vertragsstaat(en): ES, GR)

1. Verfahren zur Herstellung der Verbindungen der Formel 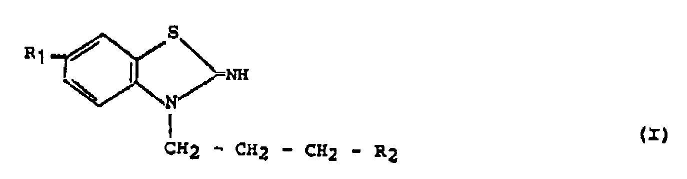 worin R₁ einen Polyfluoralkoxyrest bedeutet und R₂ einen Alkylthio-, Alkylsulfinyl- odr Alkylsulfonylrest darstellt, wobei die Alkyl- und Alkoxyteile 1 bis 4 Kohlenstoffatome in gerader oder verzweigter Kette enthalten sowie von ihren Salzen mit organischen oder Mineralsäuren, dadurch gekennzeichnet, daß man
A. zur Herstellung von Verbindungen der Formel (I), für die R₂ einen Alkylthiorest bedeutet, ein Derivat der Formel
R₃ - S Na (II)
worin R₃ einen Alkylrest darstellt, auf ein Derivat der Formel 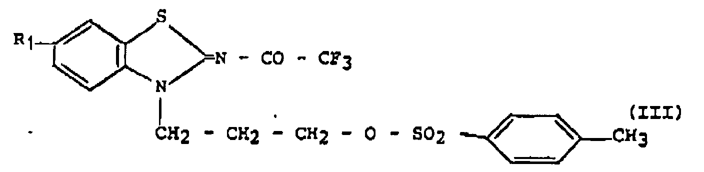 worin R₁ die gleichen Bedeutungen wie in Formel (I) hat, einwirken läßt, das Produkt dann isoliert und es gegebenenfalls in ein Additionssalz mit einer organischen oder Mineralsäure überführt,
B. zur Herstellung der Verbindungen der Formel (I), für die R₂ einen Alkylsulfinyl- oder Alkylsulfonylrest bedeutet, ein entsprechendes Derivat der Formel (I), worin R₂ einen Alkylthiorest darstellt, oxidiert und gegebenenfalls hydrolysiert, das erhaltene Produkt dann isoliert und es gegebenenfalls in ein Additionssalz mit einer organischen oder Mineralsäure überführt.

2. Verfahren gemäß Anspruch 1 zur Herstellung von Verbindungen der Formel (I), für die R₁ einen Trifluormethoxy-, Pentafluorethoxy-, 2,2,2-Trifluorethoxy- oder 1,1,2,2-Tetrafluorethoxyrest bedeutet.

## Claims (Claims for the following Contracting State(s): AT, BE, CH, DE, DK, FR, GB, IT, LI, LU NL, SE)

1. Compounds of formula: 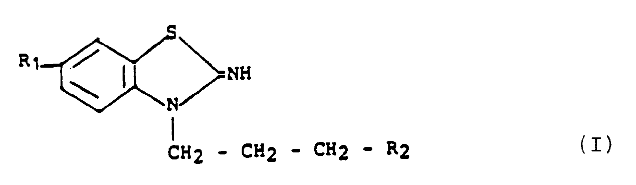 in which R₁ represents a polyfluoroalkoxy radical and R₂ represents an alkylthio, alkylsulphinyl or alkylsulphonyl radical, it being understood that the alkyl and alkoxy portions contain 1 to 4 carbon atoms in a straight or branched chain, as well as their salts with inorganic or organic acids.

2. Compounds of formula (I) according to Claim 1, for which R₁ represents a trifluoromethoxy, pentafluoroethoxy, 2,2,2-trifluoroethoxy or 1,1,2,2-tetrafluoroethoxy radical.

3. Process for the preparation of the compounds of formula (I) according to Claim 1, for which R₂ represents an alkylthio radical, characterized in that a derivative of formula:
R₃-S-Na (II)
in which R₃ represents an alkyl radical, is reacted with a derivative of formula: 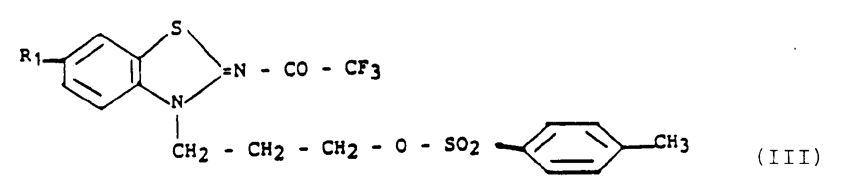 in which R₁ has the same meanings as in Claim 1, after which the product is isolated and is optionally converted to an addition salt with an organic or inorganic acid.

4. Process for the preparation of the compounds of formula (I) according to Claim 1, for which R₂ represents an alkylsulphinyl or alkylsulphonyl radical, characterized in that a corresponding derivative of formula (I), for which R₂ represents an alkylthio radical, is oxidized and, where appropriate, hydrolysed, after which the product obtained is isolated and is optionally converted to an addition salt with an organic or inorganic acid.

5. Medicaments, characterized in that they contain at least one compound according to Claim 1, or a salt of such a compound, as an active principle.

6. Medicaments according to Claim 5, for the treatment of conditions wherein glutamate is involved.

## Claims (Claims for the following Contracting State(s): ES, GR)

1. Process for the preparation of the compounds of formula: 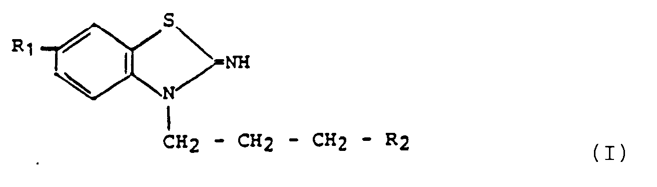 in which R₁ represents a polyfluoroalkoxy radical and R₂ represents an alkylthio, alkylsulphinyl or alkylsulphonyl radical, it being understood that the alkyl and alkoxy portions contain 1 to 4 carbon atoms in a straight or branched chain, as well as their salts with inorganic or organic acids, characterized in that
A. for the preparation of the compounds of formula (I) for which R₂ represents an alkylthio radical, a derivative of formula:
R₃-S-Na (II)
in which R₃ represents an alkyl radical, is reacted with a derivative of formula: 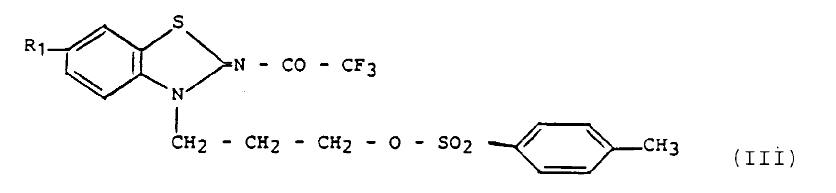 in which R₁ has the same meanings as in formula (I), after which the product is isolated and is optionally converted to an addition salt with an organic or inorganic acid,
B. for the preparation of the compounds of formula (I) for which R₂ represents an alkylsulphinyl or alkylsulphonyl radical, a corresponding derivative of formula (I), for which R₂ represents an alkylthio radical, is oxidized and, where appropriate, is hydrolysed, after which the product obtained is isolated and is optionally converted to an addition salt with an organic or inorganic acid.

2. Process according to Claim 1, for the preparation of the compounds of formula (I) for which R₁ represents a trifluoromethoxy, pentafluoroethoxy, 2,2,2-trifluoroethoxy or 1,1,2,2-tetrafluoroethoxy radical.
